(19) Europäisches Patentamt
European Patent Office
Office européen des brevets

(11) Publication number: **0 389 176 A2**

# (12) EUROPEAN PATENT APPLICATION

(21) Application number: **90302751.4**

(22) Date of filing: **15.03.90**

(51) Int. Cl.5: **C07D 501/57, C07D 499/02, C07D 471/04, C07D 498/04, //(C07D471/04,221:00,205:00), (C07D498/04,265:00,205:00)**

The title of the invention has been amended (Guidelines for Examination in the EPO, A-III, 7.3).

(30) Priority: **18.03.89 GB 8906261**
**15.08.89 GB 8918615**

(43) Date of publication of application: **26.09.90 Bulletin 90/39**

(84) Designated Contracting States: **AT BE CH DE DK ES FR GB GR IT LI LU NL SE**

(71) Applicant: **Beecham Group p.l.c.**
**SB House Great West Road**
**Brentford Middlesex TW8 9BD(GB)**

(72) Inventor: **Milner, Peter Henry**
**Brockham Park**
**Betchworth, Surrey RH3 7AJ(GB)**

(74) Representative: **Tyrrell, Arthur William Russell et al**
**SmithKline Beecham, Corporate Patents, Great Burgh, Yew Tree Bottom Road**
**Epsom, Surrey KT18 5XQ(GB)**

## (54) Beta-lactams and processes for their preparation.

(57) β-Lactams having the formula (I) or a salt thereof are described, together with a process therefor:

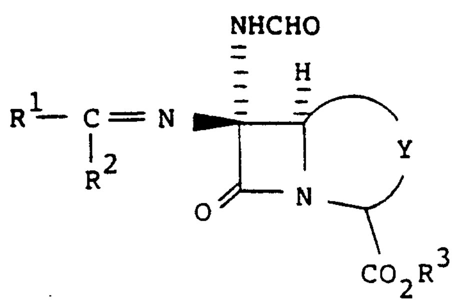

(I)

wherein Y denotes atoms completing a penicillin or cephalosporin - type nucleus, $R^1$ is a group of the formula:

HO- $[X]_n$-

in which X is an optionally substituted monocyclic or fused polycyclic aromatic ring system in which the attached hydroxy group is conjugated with the group:

EP 0 389 176 A2

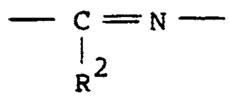

and n is 1 or 2; $R^2$ is hydrogen, $C_{1-6}$ alkyl or aryl $C_{1-6}$ alkyl; and $R_3$ is hydrogen or a carboxy protecting group.

Compounds of formula (I) are intermediates useful in the preparation of β-lactam antibiotics having an α-formamido substituent on the carbon atom adjacent to the carbonyl group of the β-lactam ring.

## Novel Compounds

This invention relates to novel $\beta$-lactam derivatives having a formamido group attached to the carbon atom adjacent to the $\beta$-lactam carbonyl group. The derivatives are useful in the preparation of $\beta$-lactam antibiotics, especially penicillins and cephalosporins, having a formamido group in that position. The invention also provides a process for the preparation of the said derivatives and a process for converting them into the said $\beta$-lactam antibiotics.

$\beta$-Lactams, for example penicillins and cephalosporins, bearing a formamido (or formamidyl) substituent adjacent to the $\beta$-lactam carbonyl group are an extremely important class of antibiotics. Such compounds are described, for example, in GB 2 107 307B and have the partial formula (A):

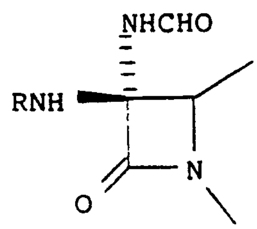

(A)

in which R is an acyl group.

Methods by which such formamido $\beta$-lactams may be prepared are described, for example, in GB 2 107 307B and European Patent Application Publication No.0 115 405.

The present invention provides a $\beta$-lactam having the formula (I) or a salt thereof:

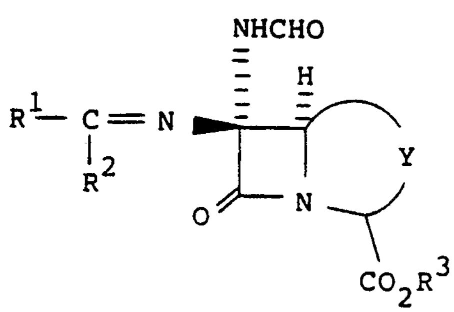

(I)

wherein $R^1$ is a group of the formula:

HO-$[X]_n$-

in which X is an optionally substituted monocyclic or fused polycyclic aromatic ring system in which the attached hydroxy group is conjugated with the group:

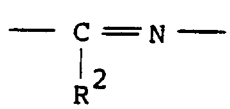

and n is 1 or 2; $R^2$ is hydrogen, $C_{1-6}$ alkyl or aryl $C_{1-6}$ alkyl; $R_3$ is hydrogen or a carboxy protecting group and Y is:

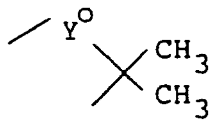 , 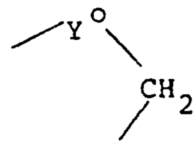

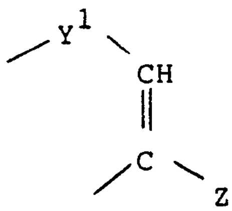 or 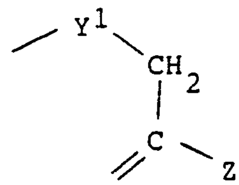 ;

wherein $Y^0$ is sulphur, SO or $SO_2$, $Y^1$ is oxygen, sulphur, SO, $SO_2$ or $-CH_2-$ and Z represents hydrogen, halogen, or an organic group such as $C_{1-4}$ alkoxy, $-CH_2Q$ or $-CH=CH-Q$ wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded via carbon, a heterocyclylthio group, or a nitrogen containing heterocyclic group bonded via nitrogen.

When X is a fused polycyclic aromatic ring system, it will be understood that the hydroxy group and the group:

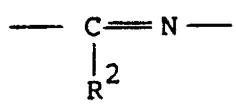

may either be attached to the same ring or attached to different rings.

When used herein the term 'halogen' refers to fluorine, chlorine, bromine or iodine.

When used herein the term "carboxylic ester" unless otherwise defined suitably includes $C_{1-6}$ alkyl esters.

When used herein the term "acyloxy" unless otherwise defined suitably includes $C_{1-6}$ alkylcarbonyloxy groups.

When used herein the term 'aryl' unless otherwise defined suitably includes phenyl and naphthyl, preferably phenyl, each optionally substituted with up to five halogen, $C_{1-6}$ alkyl, $C_{1-6}$ alkoxy, halo $(C_{1-6})$ alkyl, hydroxy, amino, carboxy, $C_{1-6}$ alkoxycarbonyl, or $C_{1-6}$ alkoxycarbonyl-$(C_{1-6})$- alkyl groups.

When used herein the term 'heterocyclyl' unless otherwise defined suitably includes single or fused rings comprising up to four hetero atoms in the ring selected from oxygen, nitrogen, and sulphur and optionally substituted with up to three halogen, $C_{1-6}$ alkyl, $C_{1-6}$ alkoxy, halo-$(C_{1-6})$-alkyl, hydroxy, amino, carboxy, $C_{1-6}$ alkoxycarbonyl, $C_{1-6}$ alkoxycarbonyl$(C_{1-6})$ alkyl, aryl, oxo, carboxy$(C_{1-6})$alkyl, carbamoyl-C-$(_{1-6})$alkyl, amino$(C_{1-6})$alkyl, mono $C_{1-6}$ alkyl amino$(C_{1-6})$ alkyl, di$(C_{1-6})$alkylamino $C_{1-6}$ alkyl, or sulphonyl$(C_{1-6})$alkyl groups.

Suitably Y is:

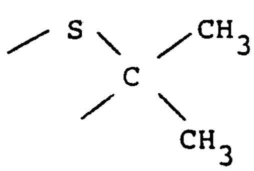 , 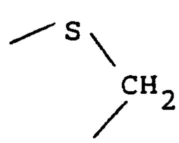 , or 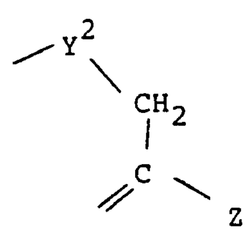

wherein $Y^2$ is oxygen, sulphur or $-CH_2-$ and Z is as hereinbefore defined.

Preferred values for Y in the compounds of formula (I) are $-S-C(CH_3)_2-$ and $-S-CH_2-C(CH_2Q)=$, ie when the compound of formula (I) is a derivative of a penicillin or cephalosporin.

In compounds of formula (I) the formamido group can exist in two preferred conformations, those wherein the hydrogen atòms of the -NH-CHO are cis or trans, of which the cis conformation normally predominates.

The group $R^1$ may be carbocylic or may contain one or more heteroatoms selected from nitrogen, oxygen and sulphur.

Suitably the group $R^1$ has one of the subformulae (a) -(e):

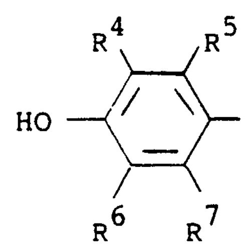

(a)

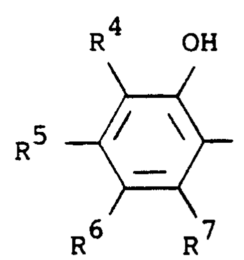

(b)

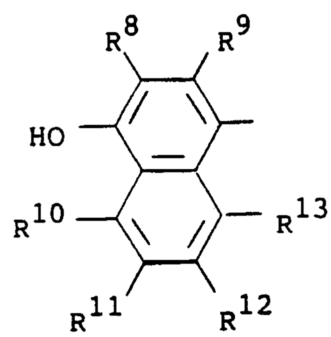

(c)

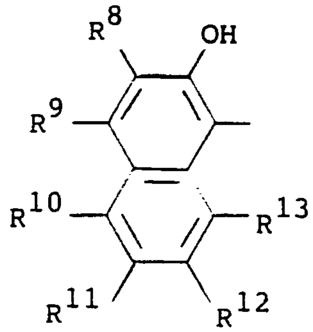

(d)

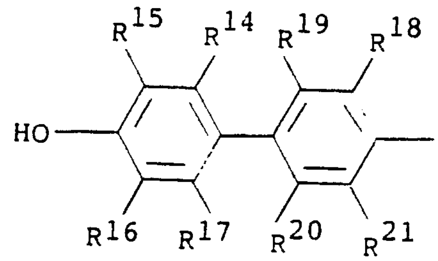

(e)

in which $R^4$ to $R^{21}$ represent organic groups which may be the same or different.

Suitable values for the groups $R^4$ to $R^{21}$ include hydrogen, halogen, alkoxy, alkylthio, cyano, nitro, carboxy, alkoxycarbonyl, carbamoyl, alkylcarbonyl, amino, mono-alkylamino, di-alkylamino, aryl, heterocyclyl, or an unsubstituted or substituted alkyl group.

Any alkyl group referred to herein may, unless otherwise indicated, be straight or branched, unsubstituted or substituted, and may contain, for example, up to 12 carbon atoms, suitably up to 6 carbon atoms.

In particular, the alkyl group may be an unsubstituted or substituted methyl, ethyl, n- propyl, iso-propyl, n-butyl, sec- butyl, isobutyl or tert-butyl group.

Examples of suitable optional substituents for any alkyl group include heterocyclyl, amino, $(C_{1-6})$-alkanoylamino, (mono, di, or tri)-$(C_{1-6})$alkylamino, hydroxy, $(C_{1-6})$alkoxy, mercapto, $(C_{1-6})$alkylthio, heterocyclylthio, arylthio, sulphamoyl, carbamoyl, amidino, guanidino, nitro, chloro, bromo, fluoro, carboxy and salts and esters thereof, $(C_{1-6})$alkanoyloxy, arylcarbonyloxy, heterocyclylcarbonyloxy and acyl groups.

In one preferred aspect the group $R^1$ has sub-formula (a) in which $R^4$ and $R^6$ are each $C_{1-6}$ alkyl and $R^5$ and $R^7$ are hydrogen.

Particular compounds of formula (I) include: t-Butyl 7β-[(3,5-di-t-butyl-4-hydroxy)benzylideneamino]-7α-formamidocephalosporanate; and p-Methoxybenzyl-7β-[(3,5-di-t-butyl-4-hydroxy) benzylideneamino]-3-chloromethyl-7α-formamidoceph-3-em-4-carboxylate.

The invention further provides a process for the preparation of a compound of formula (I):

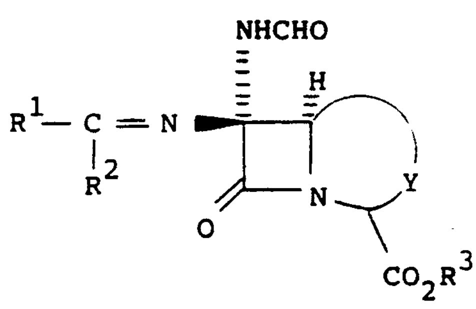

(I)

wherein $R^1$, $R^2$, $R^3$ and Y are as hereinabove defined;

which process comprises reacting a compound of formula (II) or a salt thereof:

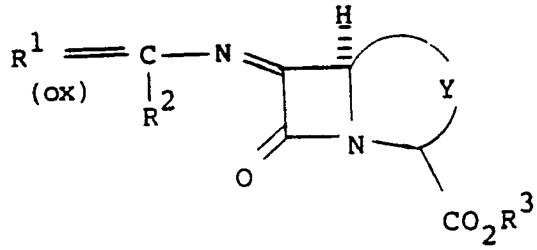

(II)

wherein $R^2$, $R^3$ and Y are as defined for formula (I), $R^1{}_{(ox)}$ is a quinone group corresponding to an oxidised form of the group $R^1$ as hereinbefore defined, and any reactive groups may be protected; with either

(a) a nucleophilic derivative of formamide;

or

(b) ammonia or a protected form thereof, and thereafter with a formylating agent; and thereafter if necessary, after step (a) or step (b), carrying out one or more of the following steps:

(i) removing any protecting groups;

(ii) converting the group $R^3$ into a different group $R^3$;

(iii) converting one group Z into a different group Z;

(iv) converting the product into a salt.

By 'protected form of ammonia' is meant a reagent which may be used to introduce a protected amino group into a compound of formula (II) so that the said protected amino group is capable of being formylated and the resulting formylated group is capable of being deprotected to give a formamido group.

Suitable protected forms of ammonia include primary amines, for example benzylamine, and silyl derivatives of ammonia.

It will be appreciated that the group $R^1{}_{(ox)}$ has the formula:

$O=[X^1]_n=$

wherein n is as hereinbefore defined and $X^1$ is an optionally substituted monocyclic or fused polycyclic ring system having an even number of double bonds each being conjugated with the carbonyl group and the exocyclic double bond, the group:

$O=[X^1]_n=$

being a quinone group corresponding to an oxidised form of the group:

$HO-[X]_n-$

as hereinbefore defined.

The group $R^1{}_{(ox)}$ suitably has one of the subformulae (f)-(j):

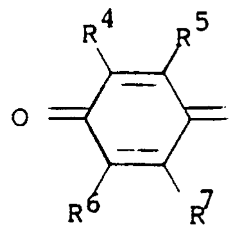

(f)

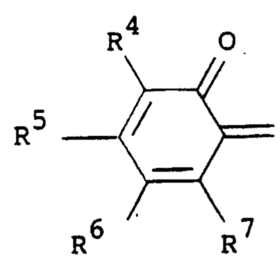

(g)

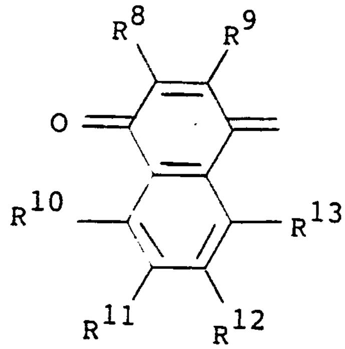

(h)

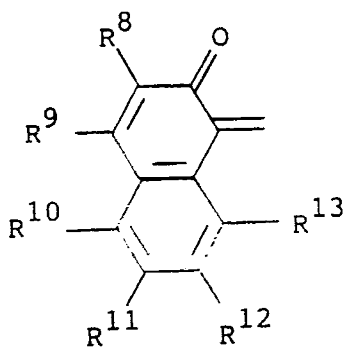

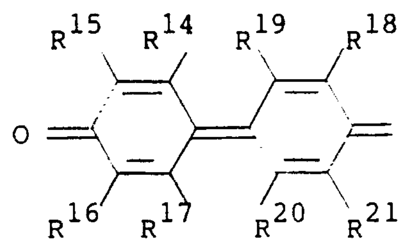

(i) (j)

wherein $R^4$-$R^{21}$ are groups as hereinabove defined.

In the compounds of formulae (I) and (II) the groups $R^1$ and $R^1_{(ox)}$ preferably have the abovementioned formula (a) or (b) and (f) or (g) respectively in which $R^4$, $R^5$, $R^6$ and $R^7$ may each represent hydrogen, $C_{1-6}$ alkyl, $C_{1-6}$ alkoxy, halogen, cyano, $C_{1-6}$ alkoxycarbonyl or $R^4$ and $R^5$ together and/or $R^6$ and $R^7$ together may form a ring fused to the benzene ring to which they are attached.

In a particularly preferred embodiment of the invention the group $R^1$ is 3,5-di-t-butyl-4-hydroxyphenyl and the group $R^1_{(ox)}$ has the corresponding quinone formula:

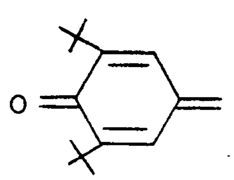

In the compounds of formulae (I) and (II) the group $R^2$ is preferably hydrogen.

Suitable nucleophilic derivatives of formamide for use in the above process include N-silyl, N-stannyl and N-phosphorylated derivatives and salts of formamide, for example the lithium salt.

The term 'N-silyl derivative' of formamide denotes the product of reaction of the amino group of formamide with a silylating agent such as a halosilane or a silazane of the formula:

$L_3$ Si.U; $L_2$ Si.$U_2$; $L_3$ Si.$NL_2$;

$L_3$ Si.NH.Si $L_3$; $L_3$ Si.NH.COL; $L_3$ Si. NH.CO.NH.Si $L_3$;

L NH.CO.NH.Si $L_3$;

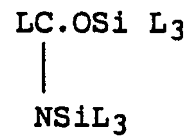

wherein U is a halogen and the various groups L which may be the same or different, each represents hydrogen or alkyl, alkoxy, aryl, or aralkyl. Preferred silylating agents are silyl chlorides, particularly trimethylchlorosilane.

The term 'N-stannyl derivative' of formamide includes the product of reaction of the amino group of formamide with a stannylating agent such as a halostannane of formula:

$L_3SnU$

wherein L and U are as defined hereinbefore.

The term 'N-phosphorylated' derivative of formamide is intended to include compounds wherein the amino group of formamide is substituted with a group of formula:

$- P.R_a\ R_b$

wherein $R_a$ is an alkyl, haloalkyl, aryl, aralkyl, alkoxy, haloalkoxy, aryloxy, aralkyloxy or dialkylamino group, $R_b$ is the same as $R_a$ or is halogen or $R_a$ and $R_b$ together form a ring.

Other suitable nucleophilic derivatives of formamide include:

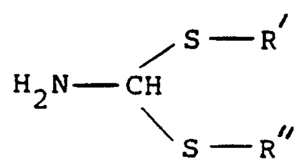

wherein $R'$ and $R''$ may be the same or different and each represent a $C_{1-6}$ alkyl group or $R'$ and $R''$ together represent a $C_{2-4}$ alkylene di-radical.

Preferably the nucleophilic derivative of formamide is a N,N-bis (tri-loweralkylsilyl) formamide, in particular N,N-bis(trimethylsilyl)formamide.

Suitable solvents in which reaction variant (a) may be performed include, for example, toluene, dioxan, tetrahydrofuran, dimethylformamide, 1,2-dichloroethane, and hexamethylphosphoramide. The reaction is generally carried out, advantageously in an inert atmosphere, at moderate to low temperatures, ie in the range -100°C to +50°C. The course of the reaction may be followed by conventional methods such as thin layer chromatography and terminated when an optimum quantity of product is present in the reaction mixture.

Optionally, the above process may be carried out in the presence of a Lewis acid.

Subsequent to the reaction it may be necessary to regenerate the formamido group from any derivative; suitable methods include those known in the art such as, for example, acid or base hydrolysis or treatment with a metal ion such as mercury, silver, thallium, lead or copper.

When the process variant (b) is used in the process for preparing a compound of the formula (I), treatment with ammonia is suitably carried out at a non-extreme temperature, for example 0-60°C, normally 10-40°C and preferably ambient. The reaction is conveniently performed in an aprotic solvent such as tetrahydrofuran or dioxan.

Suitable formylating agents for use in process variant (b) include the reagent 4-formyl-2-methyl-1,3,4-thiadiazolin-5-thione (see H. Yazawa and S. Goto, Tetrahedron Letters, 1985, 26, 3703-3706), or anhydrides such as formic acetic anhydride or formic anhydride. The reaction may suitably be carried out in a temperature in the range -50°C to 30°C in aprotic solvent such as, for example, toluene, dichloromethane, 1,2-dichloroethane, chloroform, dimethylformamide, tetrahydrofuran, hexamethylphosphoramide, or dimethylsulphoxide, in the presence of a tertiary base. A preferred tertiary base employed in the reaction is a base of the pyridine type, such as pyridine, lutidine or picoline.

Suitable readily removable carboxyl protecting groups for the group $-CO_2R^3$ in formulae (I) and (II) include ester derivatives of the carboxylic acid. The derivative is preferably one which may readily be cleaved.

Suitable ester-forming carboxyl-protecting groups are those which may be removed under conventional conditions. Such groups for $R^3$ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl, methoxymethyl, a silyl, stannyl or phosphorus-containing group, an oxime radical of formula -N = CHR$^{22}$ where R$^{22}$ is aryl or heterocyclic, or an in vivo hydrolysable ester radical such as described in European Application Publication No. 0 115 405.

The carboxyl group may be regenerated from any of the above esters by usual methods appropriate to the particular R$^3$ group, for example, acid - and base -catalysed hydrolysis, or by enzymically-catalysed hydrolysis, or by hydrogenolysis.

Suitable salts of the carboxy group of the compound of formulae (I) and (II) include metal salts eg aluminium, alkali metal salts, such as sodium or potassium, alkaline earth metal salts such as calcium or magnesium and ammonium or substituted ammonium salts.

Suitable substituted ammonium salts include quaternary ammonium salts for example optionally substituted tetraalkylammonium salts such as the trimethylbenzyl ammonium salt, or salts of the carboxy group with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylene-diamine, 1-ephenamine, N-ethylpiperidine, N-benzyl-β-phenethylamine, dehydroabietylamine, N,N′-bisdehydro-abietylamine, ethylenediamine, or bases of the pyridine type such as pyridine, collidine or quinoline, or other amines which have been used to form salts with known penicillins and cephalosporins.

Other useful salts include the lithium and silver salt.

It will be understood that, whenever necessary, conversion of one group Z into another group Z may be carried out by methods well known in the art of cephalosporin synthesis.

Removal of protecting groups may be effected by known methods which do not cause appreciable side reactions to occur elsewhere in the molecule.

Suitable values for Q in the compounds of formulae (I) and (II) include the acetoxy, heterocyclylthio group, and nitrogen containing heterocyclic group bonded via nitrogen.

More suitably Q represents the acetoxy or heterocyclylthio group.

The heterocyclylthio group may suitably be represented by the formula:

- S - Het

wherein 'Het' is a five or six membered heterocyclic ring containing from 1 to 4 atoms selected from N, O, and S unsubstituted or substituted with one or two groups selected from $C_{1-6}$ alkyl, $C_{1-6}$ alkoxy, hydroxyalkyl, $C_{1-6}$ alkenyl, alkoxyalkyl, carboxyalkyl, sulphonylalkyl, carbamoylalkyl, trifluoromethyl, hydroxy, halogen, oxo, optionally substituted aminoalkyl, and carboxy-alkyl or two substituents may be linked to form the residue of a heterocyclic or carbocyclic ring.

Examples of the group 'Het' include unsubstituted and substituted imidazolyl, triazolyl, tetrazolyl, thiazolyl, thiadiazolyl, thiatriazolyl, oxazolyl, triazinyl and oxadiazolyl.

Suitable groups 'Het' include unsubstituted and substituted 1, 2, 3-triazolyl; 1, 2, 4-triazolyl; tetrazolyl; oxazolyl; thiazolyl; 1, 3, 4-oxadiazolyl; 1, 3, 4-thiadiazolyl; or 1, 2, 4-thiadiazolyl. Preferably the heterocyclylthio group is 1-methyl-1H-tetrazol-5-ylthio, 2-methyl-1,3,4-thiadiazol-5-ylthio, 1,3,4-thiadiazol-2-ylthio, 1-carboxymethyl-1H-tetrazol-5-ylthio or 6-hydroxy-2-methyl-5-oxo-2H-1,2,4-triazin-3-ylthio.

The nitrogen containing heterocyclic group bonded via nitrogen is suitably a pyridinium group unsubstituted or substituted with one or two groups selected from $C_{1-6}$ alkyl, $C_{1-6}$ alkoxy, hydroxyalkyl, $C_{1-6}$ alkenyl, alkoxyalkyl, carboxyalkyl, sulphonylalkyl, carbamoylmethyl, carbamoyl, trifluoromethyl, hydroxy, halogen, oxo, aminoalkyl, or two substituents on adjacent carbon atoms may form the residue of a carbocyclic ring.

The compounds of formula (II) may be prepared by reacting a compound of formula (III) or a salt thereof:

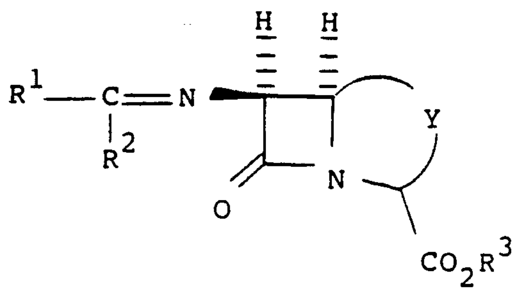

(III)

wherein $R^1$, $R^2$, $R^3$ and Y are as defined for formula (I), and any reactive groups may be protected, with an oxidising agent capable of oxidising the compound to its quinoid form.

It will be understood that the oxidising agent should not cause appreciable side-reactions elsewhere in the molecule.

Suitable oxidising agents include metal oxides, especially those of heavy or transition metals, sources of positive halogen such as hypochlorites, for example t-butyl hypochlorite, or N-bromosuccinimide, or high potential quinones such as 2,3-dichloro-5,6-dicyano-1,4-benzoquinone.

Typical oxidising agents include lead dioxide and manganese dioxide and 2,3-dichloro-5,6-dicyano-1,4-benzoquinone.

A preferred oxidising agent is 2,3-dichloro-5,6-dicyano-1,4-benzoquinone.

The reaction is suitably carried out in an inert solvent, for example aromatic hydrocarbons such as benzene or toluene; halogenated hydrocarbons such as dichloromethane or 1,2-dichloroethane; and ethers such as dioxan or tetrahydrofuran.

When 2,3-dichloro-5,6-dicyano-1,4-benzoquinone is used as the oxidising agent, a preferred solvent is dichloromethane.

The oxidising agent may be employed in a stoichiometric amount relative to the compound of formula (III) or may be used in excess. Normally an appreciable excess, for example 3-30 equivalents of oxidising agent, is used.

The reaction is suitably carried out at ambient or elevated temperature, for example 0-100°, preferably 10-80°, advantageously 20-60° C. Typically the reaction is carried out at room temperature.

If desired, the product of formula (II) may be isolated, and subsequently converted into the compound of formula (I) as hereinabove described.

Advantageously, however, the compound of formula (II) may be caused to react in situ to give a compound of formula (I), by adding an appropriate reagent, as hereinabove described, to the reaction mixture after the oxidation is substantially complete.

In the latter case, any excess of oxidising agent is normally removed by filtration before the conversion of the compound of formula (II) into the compound of formula (I) is effected.

Compounds of formula (III) are known in the art and may be prepared, conveniently in situ, by reaction of an appropriate amino-substituted β-lactam compound, for example 7β-aminocephalosporanic acid, 7β-amino-3-chloromethylceph-3-em-4-carboxylic acid, or a salt or protected derivative thereof, with an aldehyde or ketone of the general formula:

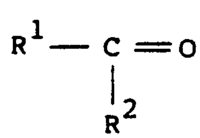

wherein $R^1$ and $R^2$ are as hereinbefore defined.

The compounds of formula (I) are useful intermediates for the preparation of β-lactam antibiotics containing a suitable acylamino group attached to the β-lactam ring in place of the group

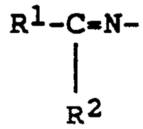

in the compounds of formula (I).

By 'suitable acylamino group' is meant a group of the formula:

$R^{23}CONH-$

where $R^{23}$ CO is an acyl group found in antibacterially active penicillins or cephalosporins.

Suitable such acyl groups include those described in GB 2 107 307B.

The compounds of formula (I) may be reacted directly with an appropriate acylating agent, or may first be reacted with a reagent capable of cleaving the C = N bond to generate a β-lactam having a free β-amino group adjacent to the β-lactam carbonyl, for example 7α-formamido-7β-aminocephalosporanic acid, or a salt or protected derivative thereof, and acylated thereafter by methods known in the art.

Reagents capable of cleaving the C = N bond in compounds of formula (I) include those known in the art to be capable of cleaving Schiff's bases.

Suitable such reagents include, for example, p-toluene sulphonic acid and 2,4-dinitrophenylhydrazine in the presence of p-toluenesulphonic acid.

One preferred reagent for the above purpose is that known as Girard's T-reagent [(carboxymethyl)-trimethylammonium chloride hydrazide] in the presence of p-toluene sulphonic acid monohydrate. Further preferred reagents are dilute mineral acid, for example dilute hydrochloric acid, and p-toluene sulphonic acid monohydrate.

The reaction may be carried out in a suitable solvent such as ethyl acetate or lower alkanol such as methanol at ambient temperature.

Accordingly, the invention further provides a process for the preparation of a β-lactam of the formula (IV) or a salt thereof:

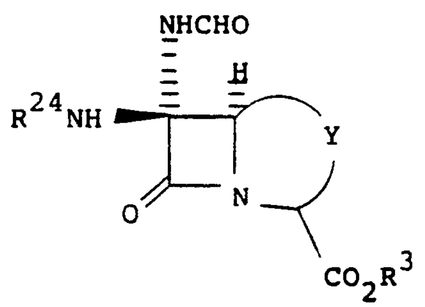

(IV)

wherein $R^3$ and Y are as hereinabove defined and $R^{24}$ is hydrogen or a group $R^{23}$ CO in which $R^{23}$ is a group found in antibacterially active penicillins or cephalosporins; which process comprises treating a compound of the formula (I), wherein any reactive groups may be protected, with a reagent capable of cleaving the C = N bond and thereafter if necessary carrying out one or more of the following steps:

i) converting the group $R^{24}$ into a different group $R^{24}$

ii) converting the group $R^3$ into a different group $R^3$;

iii) converting one group Z into a different group Z;

iv) removing any protecting groups;

v) converting the product into a salt.

Conveniently, the compound of formula (IV) may be prepared from a compound of formula (III) by the methods hereinabove described without isolating or purifying the corresponding compound of formula (II) or formula (I).

Interconversions of groups $R^{24}$, $R^3$ and Z and removal of protecting groups may be carried out by techniques known in the art, for example as described in GB 2,107,307B.

Compounds of formula (IV) which may be made by the above process include:

t-Butyl 7β-amino-7α-formamidocephalosporanate;

7β-Amino-7α-formamidocephalosporanic acid;
Sodium 7β-amino-7α-formamidocephalosporanate;
7β-Amino-7α-formamido-3-[(1,3,4-thiadiazol-2-yl)-thiomethyl]ceph-3-em-4-carboxylic acid; and
p-Methoxybenzyl 7β-amino-3-chloromethyl-7α-formamidoceph-3-em-4-carboxylate and salts thereof, for example the p-toluenesulphonic acid salt.

The following examples illustrate the invention.

Example 1

t-Butyl 7β-[(3,5-di-t-butyl-4-hydroxy)benzylideneamino]-7α-formamido cephalosooranate

a) t-Butyl 7β-[(3,5-di-t-butyl-4-hydroxy)benzylideneamino]cephalosporanate

i) Method 1

3,5-Di-t-butyl-4-hydroxybenzaldehyde (2.34g, 0.01mol) in toluene (40ml) (required warming to effect solution) was added to a stirred solution of t-butyl 7β-amino cephalosporanate (3.28g, 0.01mol) in toluene (50ml) over 3A molecular sieves (5g). The mixture was stirred at room temperature for 20h before being filtered and evaporated to dryness. The crude product was quickly chromatographed on silica gel eluting with hexane/ethyl acetate 5:1 grading to 3:1 to afford the title compound (2.86g, 53%) $\nu_{max}$ ($CH_2Cl_2$) 3630, 1780, 1738, 1725, 1635 $cm^{-1}$. δ ($CDCl_3$) 1.43 (18H, s, 2 x $C(CH_3)_3$), 1.55 (9H, s, $C(CH_3)_3$), 2.03 (3H, s, $OCOCH_3$), 3.28 and 3.51 (2H, ABq, J 18Hz, 2-$CH_2$), 4.77 and 5.02 (2H, ABq, J 12Hz, $CH_2OAc$), 5.12 (1H, d, J 5Hz, 6-H), 5.40 (1H, dd, J 5Hz, 2Hz, 7-H), 5.58 (1H, s, OH), 7.59 (2H, s, aromatics) 8.50 (1H, d, J 2Hz, CH=N).

ii) Method 2

3,5-Di-t-butyl-4-hydroxybenzaldehyde (234mg, 1mmol) and t-butyl 7β-amino cephalosporanate (328mg, 1mmol) in toluene (25 ml) were heated under reflux using a Dean and Stark apparatus. After 1h the solution was evaporated to dryness and the crude product was chromatographed on silica as above to yield the title compound (439mg, 79%).

b) t-Butyl 7β-[(3,5-di-t-butyl-4-hydroxy)benzylideneamino]-7α-formamido cephalosporanate

Preparation 1

t-Butyl 7β-[(3,5-di-t-butyl-4-hydroxy)benzylideneamino]cephalosporanate (109mg, 0.2mmol) in toluene (5ml) was treated with freshly prepared lead dioxide and the mixture was stirred at room temperature for 1h. The mixture was filtered and the filtrate was treated with N,N-bis(trimethylsilyl)formamide (189mg, 1mmol) and the reaction solution was stirred at room temperature for 4h. It was then evaported to dryness and the resulting red-brown oil was chromatographed on silica gel eluting with hexane/ethyl acetate 5:1 grading to 1:1 to afford the title compound (68mg, 58%). $\nu_{max}$ ($CH_2Cl_2$) 4610, 3395, 2950, 1780, 1740 (sh), 1725, 1695, 1630 $cm^{-1}$. Nmr showed the presence of E-(minor) and Z-(major) rotamers. δ ($CDCl_3$) 1.46 (18H, s, 2 x C-$(CH_3)_3$), 1.58 (E) and 1.60 (Z) (9H, 2s, $CO_2C(CH_3)_3$), 2.07 (Z) and 2.09 (E) (3H, 2s, $OCOCH_3$), 3.25 and 3.53 (2H, ABq, J 18.2Hz, 2-$CH_2$), 4.77 and 4.97 (2H, ABq, J 12.8Hz, $CH_2OAc$), 5.41 (1H, s, 6-H), 5.66 (Z) and 5.68 (E)) (1H, 2s, exch. $D_2O$, OH), 6.40 (ca. 0.9H, s, exch. $D_2O$, NHCHO (Z)), 6.58 (ca. 0.1H, d, J 11.8Hz, exch. $D_2O$, NHCHO (E), 7.65 (2H, s, aromatic H), 8.35 (ca. 0.9H, s, NHCHO, (Z), 8.40 (Z) and 8.46 (E) (1H, 2s, CH=N), 8.58 (ca. 0.1H, d, J 11.8Hz, s on $D_2O$, NHCHO (E)). m.s. (F.A.B.) (i) thioglycerol. $MH^+$ 588, (ii) 3-nitrobenzyl alcohol $MNa^+$ 610.

Preparation 2

t-Butyl 7β-[(3,5-di-t-butyl-4-hydroxy)benzylideneamino]cephalosporanate (1.09g, 2mmol) in toluene (25ml) was treated with freshly prepared dry lead dioxide (1.58g, 6.6mmol) and the reaction mixture was stirred at room temperature for 1h. It was then filtered, and the filtrate was treated with N,N-bis-(trimethylsilyl)formamide (2.13ml, 10mmol) and the reaction solution was stirred at room temperature for 3h. The solution was evaporated to dryness and the crude product was chromatographed on silica gel eluting with hexane/ethyl acetate 5:1 grading to 1:1 to afford the title compound (112mg, 10%) plus t-butyl 7β-[(3,5-di-t-butyl-4-(trimethylsilyl)oxy)benzylideneamino]-7α-formamido cephalosporanate (281mg, 21%). $\nu_{max}$ - ($CH_2Cl_2$) 3395, 2950, 1780, 1740 (sh), 1725, 1695, 1630 $cm^{-1}$. δ ($CDCl_3$) 0.42 (9H, s, $Si(CH_3)_3$), 1.42 (18H, s, 2 x $C(CH_3)_3$), 1.60 (9H, s, $CO_2C(CH_3)_3$), 2.08 (3H, s, $OCOCH_3$), 3.25 and 3.53 (2H, ABq, J 18.2Hz, 2-$CH_2$), 4.78 and 4.96 (2H, ABq, J 12.8Hz, $CH_2OAc$), 5.42 (1H, s, 6-H), 6.34 (1H, s, NHCHO), 7.72 (2H, s, aromatic H), 8.35 (1H, s, NHCHO) and 8.42 (1H, s, CH=N). m.s. (F.A.B.) 3-nitrobenzyl alcohol 660 $MH^+$, 682 $MNa^+$. This trimethylsilyloxy derivative could readily be converted to the title compound by treatment with tetrabutyl ammonium fluoride in methanol.

Example 2

t-Butyl 7β-amino-7α-formamido cephalosporanate

t-Butyl 7β-[(3,5-di-t-butyl-4-hydroxy)benzylideneamino]-7α-formamido cephalosporanate (41mg, 0.07mmol) in methanol (3ml) was treated with Girard's reagent T (26mg, 0.154mmol) and p-toluene sulphonic acid monohydrate (115mg, 0.077mmol). After 0.5h the reaction solution was evaporated to dryness and the crude product was dissolved in ethyl acetate and washed with dilute aqueous sodium hydrogen carbonate, water and brine. After drying over magnesium sulphate, the organic solution was filtered and evaporated to dryness to afford an off-white solid. This was chromatographed on silica gel eluting with hexane/ethyl acetate 1:3 to yield the title compound (14mg, 54%). $\nu_{max}$ ($CH_2Cl_2$) 3410, 1790, 1740, 1700 $cm^{-1}$. δ ($CDCl_3$) 1.58 (9H, s, $C(CH_3)_3$), 2.10 (3H, s, $OCOCH_3$), 2.43 (2H, s, $NH_2$), 3.35 and 3.55 (2H, ABq, J 18Hz, 2-$CH_2$), 4.81 and 4.99 (2H, ABq, J 13Hz, $CH_2OAc$), 5.11 (1H, s, 6-H), 6.92 (1H, s, NHCHO), 8.25 and 8.26 (1H, 2s, CHO).

Example 3

t-Butyl 7β-[(3,5-di-t-butyl-4-hydroxy)benzylideneamino]-7α-formamido cephalosporanate

t-Butyl 7β-[(3,5-di-t-butyl-4-hydroxy)benzylideneamino]cephalosporanate (86mg, 0.16mmol) in toluene (5ml) was treated with manganese dioxide (280mg, 3.2mmol) in three portions over 1h. The mixture was filtered and N,N-bis(trimethylsilyl)formamide (151mg, 0.8mmol) was added and the solution was stirred at room temperature for 16h. The solution was evaporated to dryness and the crude product was chromatographed on silica eluting with hexane/ethyl acetate 5:1 grading to 1:1 to afford the title compond, (12mg, 13%).

Example 4

t-Butyl 7-[(3,5-di-t-butyl-4-oxo-2,5-cyclohexadien-1-ylidene)methyleneimino]cephalosporanate

t-Butyl 7β-[(3,5-di-t-butyl-4-hydroxy)benzylideneamino]cephalosporanate (109mg, 0.2mmol) in toluene (5ml) was treated with freshly prepared lead dioxide (239mg, 1.0mmol) and the mixture was stirred at room temperature for 1h. The mixture was filtered and the filtrate was evaporated to dryness. The crude product was quickly chromatographed on silica gel eluting with hexane/ethyl acetate 5:1 grading to 3:1 to afford the title compound (61mg, 56%). $\nu_{max}$ ($CH_2Cl_2$) 2950, 1770, 1735, 1725, 1680, 1610 $cm^{-1}$. δ ($CDCl_3$) 1.30 (9H, s, $C(CH_3)_3$), 1.33 (9H, s, $C(CH_3)_3$) 1.60 (9H, s, $CO_2C(CH_3)_3$), 2.12 (3H, s, $OCOCH_3$), 3.45 and 3.65 (2H,

ABq, J 18.5Hz, 2-$CH_2$), 4.84 and 5.10 (2H, ABq,J 13Hz, $CH_2OAc$), 5.39 (1H, br.s, 6-H), 6.98 (1H, br.s, $CH=\overset{+}{N}$), 7.90 (1H, br.s, cyclohexadienyl H) 8.04 (1H, br.s, cyclohexadienyl H). $C_{29}H_{38}N_2O_6S$ requires $M^+$ 542.2451, Found $M^+$ 542.2442.

Example 5

7β-Amino-7α-Formamido cephalosporanic acid

7β-Amino cephalosporanic acid (680mg, 2.5mmol) was suspended in dry dichloromethane (15ml) and triethylamine (1.74ml, 12.5mmol) was added. The resulting solution was stirred at room temperature for 5 minutes before being evaporated to dryness. The residue was treated with toluene and re-evaporated. After evaporating once more from dichloromethane the residue was dried under high vacuum. The resulting foam was dissolved in dry dichloromethane (15ml) and treated with chlorotrimethylsilane (0.35ml, 2.75mmol) and then stirred at room temperature under argon for 0.5h. The reaction solution was then evaporated to dryness, the residue was treated with dry tetrahydrofuran and the resulting mixture was filtered. The filtrate was evaporated to dryness, the residue was dissolved in dry dichloromethane (25ml) and then treated with 4A molecular sieves followed by 3,5-di-t-butyl-4-hydroxybenzaldehyde (585mg, 2.5mmol). The reaction mixture was stirred at room temperature under argon for 16h, and then treated with a solution of 2,3-dichloro-5,6-dicyano-1,4-benzoquinone (567mg, 2.5mmol) in dichloromethane (5ml) and N,N-dimethylformamide (1ml). After stirring for 1h at room temperature under argon the mixture was filtered and the filtrate was treated with N,N-bis(trimethylsilyl)formamide (1.42g, 7.5mmol). After stirring under argon at room temperature for 5h. the reaction solution was evaporated to dryness, and the residue was re-dissolved in methanol (30ml) before being treated with 1N hydrochloric acid (2ml). After standing at room temperature for 5 minutes the solution was evaporated to near dryness and ethyl acetate and water were added and the pH was adjusted to 6.5 with aqueous sodium hydrogen carbonate. The two phases were separated and the aqueous phase was washed with ethyl acetate (x2) before being freeze-dried to afford the crude product. This was dissolved in water and passed down a column of Diaion HP20SS resin (Mitsubishi Chem. Co.) eluting with water to afford the pure sodium 7β-amino-7α-formamido cephalosporanate (77mg, 9%). This material had identical hplc characteristics to those of authentic 7β-amino-7α-formamido cephalosporanic acid. $\nu_{max}$ (KBr) 1759, 1671, 1608, 1399, 1233cm$^{-1}$. δ (TFA) 2.30 (3H, s, $OCOCH_3$), 3.77 and 3.85 (2H, ABq, J 12Hz, 2-$CH_2$), 5.33 and 5.50 (2H, ABq, J 14.5Hz, $CH_2OAc$), 5.47 (1H, s, 6-H), 8.58 and 8.68 (1H, 2s, CHO rotamers).

Example 6

7β-Amino-7α-Formamido cephalosporanic acid

7β-Amino cephalosporanic acid (680mg, 2.5mmol) was suspended in dry dichloromethane (15ml) and treated with N,O-bis(trimethylsilyl)acetamide (560mg, 2.75mmol) followed by chlorotrimethylsilane (1 drop). The mixture was stirred at room temperature under an atmosphere of argon for 1h. after which time complete solution had occurred. 4A molecular sieves were then added followed by 3,5-di-t-butyl-4-hydroxybenzaldehyde (585mg, 2.5mmol), and the resulting mixture was stirred at room temperature under argon for 22h. A solution of 2,3-dichloro-5,6-dicyano-1,4-benzoquinone (567mg, 2.5mmol) in dichloromethane (3ml) and N,N-dimethylformamide (2ml) was then added and the mixture was stirred at room temperature for 0.5h. The mixture was filtered through a glass fibre pad, and then the filtrate was treated with N,N-bis(trimethylsilyl)formamide (1.42g, 7.5mmol) and stirred under argon at room temperature for 6h. The reaction solution was then evaproated to dryness, and the residue was dissolved in methanol (30ml) and water (5ml) and the pH was adjusted to 2 with 1N hydrochloric acid. The solution was stirred for 15 minutes before being evaporated to near dryness and then treated with ethyl acetate and water. The pH was adjusted to 6.5 with dilute aqueous sodium hydrogen carbonate, and the two phases were separated. The aqueous phase was washed with ethyl acetate (x2) before being freeze-dried to afford a brown foam. This was purified on Diaion HP20SS resin eluting with water to afford the title compound (243mg, 29%), identical in hplc and spectral characteristics to authentic material.

Example 7

7β-Amino-7α-formamido cephalosporanic acid

7β-Amino cephalosporanic acid (680mg, 2.5mmol) was suspended in dry dichloromethane (15ml) and treated with N-methyl-N-(trimethylsilyl)trifluoroacetamide (MSTFA) (547mg, 2.75mmol) and chlorotrimethylsilane (1 drop). After stirring under argon for 2.5h complete solution had not been attained so further MSTFA (275mg) was added and the mixture was heated under reflux in an argon atmosphere for 15 minutes. The resulting solution was then evapoated to dryness, and the residue was heated under vacuum at ca. 50 °C for 0.5h. This was then dissolved in dry dichloromethane (20ml) and 4A molecular sieves followed by 3,5-di-t-butyl-4-hydroxybenzaldehyde (585mg, 2.5mmol) were added, and the mixture was stirred under argon at room temperature for 16h. A solution of 2,3-dichloro-5,6-dicyano-1,4-benzoquinone (567mg, 2.5mmol) in dichloromethane (3ml) and N,N-dimethylformamide (1ml) was then added, and the reaction mixture was stirred at room temperature for 0.5h. It was then filtered and the filtrate was treated with N,N-bis(trimethylsilyl)formamide (1.42g, 7.5mmol), and stirred under argon for 5h. The reaction solution was then evaporated to dryness and the residue was dissolved in methanol (20ml) and water (5ml) and adjusted to pH 2 with 1N hydrochloric acid. After stirring at room temperature for 15 minutes the solution was evaporated to near dryness, and the residue was dissolved in ethyl acetate and water and adjusted to pH 6.5 with dilute aqueous sodium hydrogen carbonate. The two phases were separated and the aqueous phase was washed with ethyl acetate (x2) and then freeze-dried. The resulting orange coloured material was purified by passage through a column of Diaion HP20SS resin eluting with water, to afford the title compound as its sodium salt (247mg, 29%). This was identical in hplc and spectral characteristics to authentic material.

Example 8

t-Butyl 7β-[(3,5-di-t-butyl-4-hydroxy)benzylideneamino]-7α-formamidocephalosporanate

t-Butyl 7β-[(3,5-di-t-butyl-4-hydroxy)benzylideneamino]cephalosporanate (0.5g, 0.918 m mole) in dichloromethane (5ml) was treated with 2,3-dichloro-5,6-dicyano-1,4-benzoquinone (DDQ) (0.21g, 0.925 m mole). The dark red solution was stirred at ambient temperature for 50 minutes. The resulting precipitate of 2,3-dichloro-5,6-dicyanohydroquinone ($DDQH_2$) was filtered off (using Hyflo supercel) and the residue was washed with dichloromethane (15ml). The combined filtrates were stirred with N,N-bis(trimethylsilyl)-formamide (0.59g, 3.12 m mole) at ambient temperature for 2 hours. The solution was concentrated under reduced pressure to a red-brown oil which was then chromatographed on silica gel. Elution with hexane/ethyl acetate 5:1 grading to 1:1 gave the title compound (0.34g, 63%).

Example 9

7α-Formamido-7β-aminocephalosporanic acid

7β-Aminocephalosporanic acid (30g, 0.11mol) was suspended in MDC (30ml) and treated with N,O-bis-(trimethylsilyl)acetamide (BSA) (30ml, 0.12mol). The suspension was stirred at ambient temperature for 60 mins until a light green translucent solution was formed. 3,5-Di-t-butyl-4-hydroxybenzaldehyde (26.5g, 0.113mol) was added and stirring continued at ambient temperature for 23 hours.

2,3-Dichloro-5,6-dicyano-1,4-benzoquinone (DDQ) (25g, 0.11mol) was added to the cooled (10 °C) orange-brown suspension. The solids dissolved to give a much darker solution. Cooling was removed after 10 mins and the reaction allowed to warm to room temperature. A fine precipitate of off-white hydro-quinone formed and this was filtered off after 1 hour. The cake was washed with MDC (100ml) and the combined filtrates treated with N,N-bis(trimethylsilyl)formamide (BSF) (62g, 0.33mol). The solution was stirred for 3 hours at ambient temperature then cooled to 5 °C before the slow addition of water (15ml, 0.83mol) and glacial acetic acid (1ml). The precipitate formed was collected after 10 mins. The solid was re-suspended in MDC (400ml) and stirred for 20 mins. It was then filtered. The residue was re-suspended in THF (230ml)

stirred for 30 mins then filtered, washed with THF (100ml) and sucked dry. The product was dried in the fan oven. Yield 17.9g. Assayed by HPLC at 94% giving an activity yield of 7α-formamido-7β-aminocephalosporanic acid of 48.4%.

Example 10

7β-Amino-7α-formamido-3-[(1,3,4-thiadazol-2-yl)thiomethyl]ceph-3-em-4-carboxylic acid

A suspension of 7β-amino-3-[(1,3,4-thiadazol-2-yl)thiomethyl]ceph-3-em-4-carboxylic acid (0.825g, 2.5mmol) in dry dichloromethane (20ml) was treated with N,O-bis(trimethylsilyl)acetamide (0.56g, 2.75mmol) and the mixture was stirred under argon at room temperature for 1.5h. The resulting solution was then treated with 4A molecular sieves followed by 3,5-di-t-butyl-4-hydroxybenzaldehyde (0.585g, 2.5mmol), and the mixture was stirred at room temperature under argon for 20h. A solution of 2,3-dichloro-5,6-dicyano-1,4-benzoquinone (0.567g, 2.5mmol) in a mixture of dichloromethane (3ml) and N,N-dimethylformamide (1ml), was then added, and the reaction mixture was stirred for 0.75h. It was then filtered under an argon atmosphere, and N,N-bis(trimethylsilyl)formamide (1.42g, 7.5mmol) was added to the filtrate. The resulting red-brown solution was stirred at room temperature under an argon atmosphere for 5h. It was then stored overnight at 5°C before being evaporated to dryness.. The resulting residue was dissolved in a mixture of methanol (20ml) and water (5ml) and the solution was treated with 1M hydrochloric acid to pH 1.5. After standing for 0.25h the solution was evaporated to dryness, then re-dissolved in a mixture of ethyl acetate and water before being adjusted to pH 6.5 with dilute aqueous sodium hydrogen carbonate solution. The two phases were separated and the aqueous phase was washed with ethyl acetate (x2) before being freeze dried to afford the crude title compound. Purification by passage through a column of Diaion HP20SS resin eluting with water, afforded the pure title compound as its sodium salt (0.092g). This material was identical in HPLC and spectral characteristics to authentic material. $\nu_{max}$ (KBr) 1758, 1670, 1603, 1525cm$^{-1}$. δ ($D_2O$) 3.43 and 3.75 (2H, ABq, J 17.4Hz, 2-$CH_2$), 4.03 and 4.43 (2H, ABq, J 13.8Hz, 3′-$CH_2$), 5.13 (1H, s, 6-H), 8.16 (1H, s, CHO), 9.41 (1H, s, thiadiazolyl-H). m.s. (F.A.B.) DMSO/glycerol/thioglycerol $MH^+$ 396.

Example 11

p-Methoxybenzyl-7β-[(3,5-di-t-butyl-4-hydroxy) benzylideneamino]-3-chloromethyl-7α-formamidoceph-3-em-4-carboxylate

a) p-Methoxybenzyl-7β-amino-3-chloromethyl-ceph-3-em-4-carboxylate hydrochloride (hereinafter called 7 ACLE-HCl)

This starting material may be prepared by the method of S. Torii et al., Tetrahedron Lett. 23, 2187 (1982) or by methods in the references cited therein.

The material may also be purchased from Otsuka Chemical Co. Ltd., Osaka, Japan.

b) p-Methoxybenzyl-7β-[(3,5-di-t-butyl-4-hydroxy) benzylideneamino]-3-chloromethyl-7α-formamidoceph-3-em-4-carboxylate

7-ACLE-HCl (4.05g; 0.01 mole) was suspended with stirring in ethyl acetate (20cm$^3$). Saturated aqueous sodium bicarbonate (20cm$^3$) was added and the stirring continued for 10 minutes. The phases were separated and the aqueous layer was re-extracted with ethyl acetate (10cm$^3$). The combined organic extracts were washed with brine (20cm$^3$) and dried (anhydrous magnesium sulphate). Evaporation under reduced pressure gave a white foam which was redissolved in dry dichloromethane (50cm$^3$). To this stirred solution at room temperature was added 3,5-di-t-butyl-4-hydroxybenzaldehyde (2.65g; 0.0113 mole). The solution was allowed to stand at room temperature overnight protected from atmospheric moisture. The solvent was removed under reduced pressure and the resultant dark oil azeotroped, in vacuo, with toluene (2 x 30cm$^3$). The crude material was re-dissolved in dichloromethane (100cm$^3$) and cooled to 5°C (ice

bath). DDQ (2,3-dichloro-5,6-dicyano-1,4-benzoquinone) (2.5g; 0.011 mole) was added to the stirred solution at 5° C. The solid eventually dissolved to give a dark solution. After 5 minutes at 5° C the reaction mixture was allowed to warm to room temperature and stirred for a further 60 minutes. During this time a light coloured precipitate appeared (DDQ$H_2$). The solid was removed by filtration, the residue being well washed with dichloromethane. To the stirred filtrates at room temperature was added bis-trimethylsilyl-formamide (6.2g; 0.033 mole). Stirring was continued for a further 2 hours. The solution was washed with water (2 x 100cm$^3$), brine (100cm$^3$) and dried (anhydrous magnesium sulphate). Evaporation of the solvent under reduced pressure gave a dark oil. This was chromatographed on silica gel (100g), eluting with 50% ethyl acetate/n-hexane, to give 2.3g (38%) of the title compound as a yellow foam.

250 MHz NMR ($CDCl_3$) δppm.

1.37 (18, bs, 2x($(CH_3)_3$); 3.40 (2H, ABq, S-$CH_2$); 3.72 (3H, s, $OCH_3$); 4.29 (2H, d, J=2.4Hz, $CH_2Cl$); 5.24 (2H, s, $CO_2CH_2$-); 5.35 (1H, s, $C_6H$); 5.60 and 5.62 (1H, 2s, $D_2O$ exchange, Z+E 4-OH); 6.59 (1H, s, $D_2O$ ex. NHCHO); 7.08 (4H, ABq, $CH_2$Ph); 7.56 (2H, s, 2, 6Ph); 8.25 (1H, s, NHCHO); 8.30 and 8.37 (1H, s+m, Z+E, CH=N).

## EXAMPLE 12

p-Methoxybenzyl 7β-amino-3-chloromethyl-7α-formamidoceph-3-em-4-carboxylate.p-toluenesulphonic acid salt

p-Methoxybenzyl 7β-amino-3-chloromethylceph-3-em-4-carb oxylate.hydrochloride (2g, 4.9mmol) was suspended in ethyl acetate (25ml) and the stirred suspension treated with saturated aqueous sodium hydrogen carbonate (25ml) until a clear solution was obtained. The two phases were separated, the aqueous extracted with ethyl acetate (10ml) and the combined organics washed with saturated brine (20ml). The separated organic solution was dried over $MgSO_4$, filtered and the stirred filtrate reacted with 3,5-di-t-butyl-4-hydroxybenzaldehyde (1.26g, 5.4mmol). Gentle warming of this mixture resulted in complete dissolution of solids. This clear solution was stirred for 1.5hrs before the solvent was evaporated under reduced pressure. Toluene (sodium dried, 30ml) was added to the residue and the solvent re-evaporated; this procedure was repeated (3x) to give an oil.

The oil was dissolved in dry dichloromethane (40ml), the solution stirred at ambient temperature and 2,3-dichloro-5,6-dicyano-1,4-benzoquinone (1.2g, 5.3mmol) added. After 1.5hrs, the mixture was filtered, washing with more dry dichloromethane (25ml) and the combined filtrates reacted at ambient temperature with N,N-bis-trimethylsilylformamide (3g, 15.8mmol) for 1hr. The solvent was then evaporated and the residue dissolved in ethyl acetate (65ml), treated sequentially with water (0.65ml) and p-toluene sulphonic acid monohydrate (0.86g, 4.5mmol) in ethyl acetate (15ml). This gave the title comoound as an off-white solid (mpt.99° C - decomp.) which was filtered off, washed with ethyl acetate followed by diethyl ether. The salt (1.91g, 67%) exhibited the following spectroscopic characteristics; $\nu_{max.}$ (KBr) 3220, 3170, 2840, 1795, 1725, 1685, and 1154cm$^{-1}$, δ[$(CD_3)_2SO$] (major rotamer only) 2.29 (3H, s), 3.65 and 3.76 (2H, ABq, J 17.7Hz), 3.76 (3H, s), 4.00 br (3H), ($D_2O$ exch.), 4.44 and 4.54 (2H, ABq, J 11.5Hz), 5.19 and 5.31 (2H, ABq, J 12.1Hz), 5.32 (1H, s), 6.94 and 7.39 (each 2H, each d, J 8.7Hz), 7.11 and 7.47 (each 2H, each d, J 8.0Hz), 8.20 (1H, s), and 10.24 br (1H, s) ($D_2O$ exch.).

## EXAMPLE 13

p-Methoxybenzyl 7β-amino-3-chloromethyl-7α-formamidoceph-3-em-4-carboxylate

p-Methoxybenzyl 7β-amino-3-chloromethyl-7α-formamidoceph-3-em-4-carboxylate.p-toluenesulphonic acid salt (583mg, 1mmol) was suspended in ethyl acetate (25ml) and the stirred suspension treated with saturated aqueous sodium hydrogen carbonate (25ml) for 30min. The resulting two phases were separated and the aqueous re-extracted with ethyl acetate (5ml). The combined organic extracts were dried and evaporated to give an orange brown solid which was triturated with acetone. The resulting solid was filtered off, washed with more acetone and ether to give the title compound (144mg, 35%) as an off-white powder.

Evaporation of the liquors and silica gel column chromatography of the residue (eluting with hexane/ethyl acetate gradient) afforded a further quantity (160mg, 39%) of the desired amine. The solid was

recrystallised from boiling ethyl acetate (m.p. 151 °C) and gave the following spectral characteristics; $\nu_{max.}$ - (KBr) 3370, 3280, 3140, 2951, 1795, 1710, 1680, 1630, and 1615cm$^{-1}$, $\delta[(CD_3)_2SO]$ (major rotamer only) 3.35 br (2H) ($D_2O$ exch.) 3.45 and 3.70 (together 2H, ABq, J 17.6Hz), 3.75 (3H, s), 4.38 and 4.44 (together 2H, ABq, J 11.4Hz), 5.15 (1H, s), 5.17 and 5.28 (together 2H, ABq, J 12.2Hz), 6.29 and 7.39 (each 2H, each d, J 8.7Hz), 8.05 (1H, d, J 1.1Hz) (changes to s on $D_2O$ exch.) and 9.02 br (1H, s); m/z (positive xenon F.A.B.; 3-nitrobenzyl alcohol - sodium acetate) $MNa^+$, 434. Found: C, 49.80; H, 4.41; N, 10.00; S, 7.64. $C_{17}H_{18}N_3O_5SCl$ requires C, 49.57; H, 4.40; N, 10.20; S, 7.79%.

## EXAMPLE 14

p-Methoxybenzyl 7$\beta$-amino-3-chloromethyl-7$\alpha$-formamidoceph-3-em-4-carboxylate.p-toluenesulphonic acid salt

a) p-Methoxybenzyl-7$\beta$-[(3,5-di-tert-butyl-4-hydroxy)benzylideneaminol-3-chloromethylceph-3-em-4-carboxylate

To a stirred suspension of p-methoxybenzyl 7$\beta$-amino-3-chloromethylceph-3-em-4-carboxylate hydrochloride (10g, 24.7mmol) in ethyl acetate (175ml) was added saturated aqueous sodium hydrogen carbonate (125ml), the mixture stirred vigorously for 40 minutes and then separated. The aqueous phase was extracted with fresh ethyl acetate (50ml). The combined organic extracts were washed with saturated brine (100ml), dried ($MgSO_4$) and filtered. The filtrate was treated with 3,5-di-tert-butyl-4-hydroxybenzaldehyde (6g, 25.6mmol) (a little warming was necessary to effect complete dissolution of solids) and the resultant solution stirred at room temperature for 16hrs before being evaporated under reduced pressure. The residue was dissolved in sodium-dried toluene (150ml) and the solution re-evaporated; this process was repeated (3x) to give an oily residue. This was chromatographed over silica gel (eluting with di-iso-propyl ether) to give an off-white solid which was crystallised from ethyl acetate-hexane to afford the title compound (10.75g, 74%) as a white solid, m.pt 176 °C (decomp.), which exhibited the following spectroscopic characteristics; $\nu_{max.}$ (KBr) 3615, 3440, 2955, 1780, 1730, 1630, 1610 and 1585cm$^{-1}$, $\delta[CDCl_3]$ 1.45 (18H, s), 3.41 and 3.65 (2H, ABq, J 18.2Hz), 3.82 (3H, s), 4.38 and 4.57 (2H, ABq, J 11.8Hz), 5.14 (1H, d, J 5.2Hz), 5.25 (2H, s), 5.41 (1H, dd, J 5.2 and 1.4Hz), 05.57 (1H, s) ($D_2O$ exch.), 6.91 and 7.38 (each 2H, each d, J 8.6Hz), 7.61 (2H, s), and 8.51 (1H, d, J 1.4Hz); m/z (positive xenon F.A.B.; thioglycerol) $MH^+$ 585. (positive xenon F.A.B.; 3-nitrobenzyl alcohol - sodium acetate) $MH^+$ 585, $MNa^+$ 607. Found: C, 63.66; H, 6.38; N, 4.89. $C_{31}H_{37}N_2SO_5Cl$ requires C, 63.64; H, 6.33; N, 4.79%.

b) p-Methoxybenzyl 7$\beta$-amino-3-chloromethyl-7$\alpha$-formamidoceoph-3-em-4-carboxylate.p-toluenesulohonic acid salt

To a solution of p-methoxybenzyl-7$\beta$-[(3,5-di-tert-butyl-4-hydroxy)benzylideneamino]-3-chloromethylceph-3-em-4-carboxylate (1g, 1.7mmol) in dry dichloromethane (15ml) at ambient temperature was added 2,3-dichloro-5,6-dicyano-1,4-benzoquinone (0.4g, 1.7mmol), the mixture stirred for 1.5hrs and then the solid which had formed during this time was filtered off. The filtrate was treated with N,N-bis-trimethylsilylformamide (0.97g, 5.14mmol) for 1hr. The solvent was evaporated, the residue dissolved in ethyl acetate (25ml) and then treated with water (0.2ml). Upon addition of a solution of p-toluene sulphonic acid monohydrate (0.33g, 1.7mmol) in ethyl acetate (5ml) to the above mixture, the title compound was produced as an off-white solid which was filtered off, washed with ethyl acetate followed by diethyl ether and then dried in vacuo. Yield = 0.77g, 77%. The product possessed spectroscopic characteristics identical to that shown by the product in Example 12.

## Claims

1. A $\beta$-lactam having the formula (I) or a salt thereof:

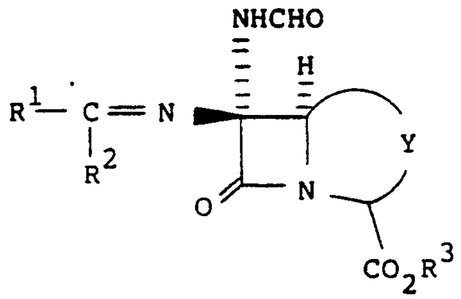

(I)

wherein $R^1$ is a group of the formula:

HO-$[X]_n$-

in which X is an optionally substituted monocyclic or fused polycyclic aromatic ring system in which the attached hydroxy group is conjugated with the group:

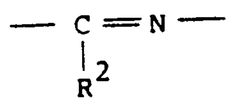

and n is 1 or 2; $R^2$ is hydrogen, $C_{1-6}$ alkyl or aryl $C_{1-6}$ alkyl; $R^3$ is hydrogen or a carboxy protecting group and Y is:

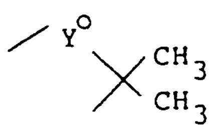

,

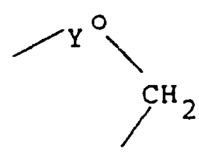

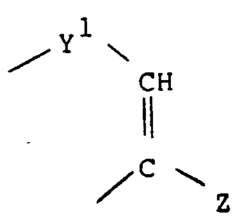

or

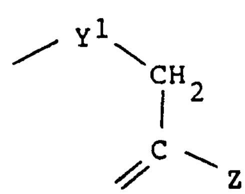

;

wherein $Y^0$ is sulphur, SO or $SO_2$, $Y^1$ is oxygen, sulphur, SO, $SO_2$ or $-CH_2-$ and Z represents hydrogen, halogen, $C_{1-4}$ alkoxy, $-CH_2Q$ or $-CH=CH-Q$ wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded via carbon, a heterocyclylthio group, or a nitrogen containing heterocyclic group bonded via nitrogen.

2. A compound as claimed in Claim 1 wherein Y is:

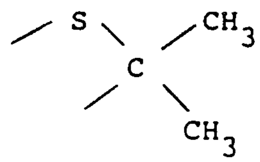 , 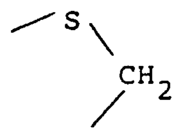 , or 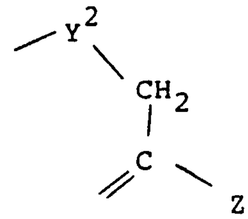

wherein $Y^2$ is oxygen, sulphur or $-CH_2-$ and Z is as defined in Claim 1.

3. A compound as claimed in Claim 1 or Claim 2 wherein the group $R^1$ has one of the subformulae (a) -(e):

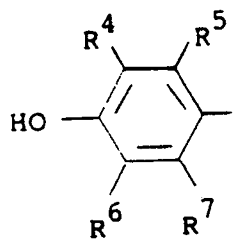

(a)

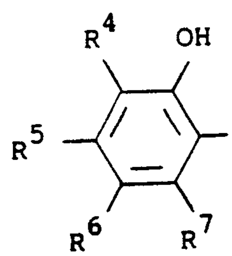

(b)

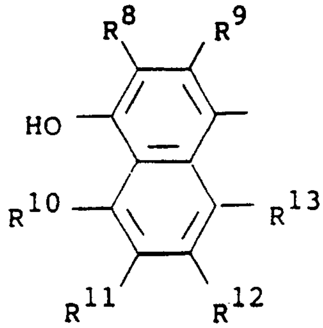

(c)

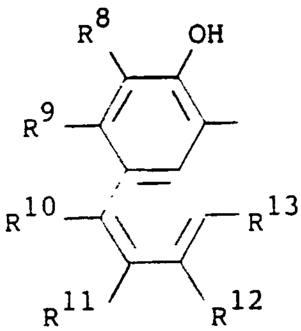

(d)

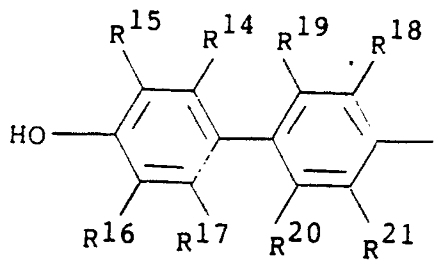

(e)

in which $R^4$ to $R^{21}$ are the same or different and each is hydrogen, halogen, alkoxy, alkylthio, cyano, nitro, carboxy, alkoxycarbonyl, carbamoyl, alkylcarbonyl, amino, mono-alkylamino, di-alkylamino, aryl, heterocyclyl, or an unsubstituted or substituted alkyl group.

4. A compound as claimed in any one of Claims 1 to 3 in which $R^2$ is hydrogen.

5. A compound as claimed in Claim 4 wherein the group:

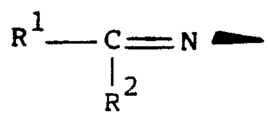

is (3,5-di-t-butyl-4-hydroxy)benzylideneamino.

6. A process for the preparation of a compound of formula (I) or a salt thereof:

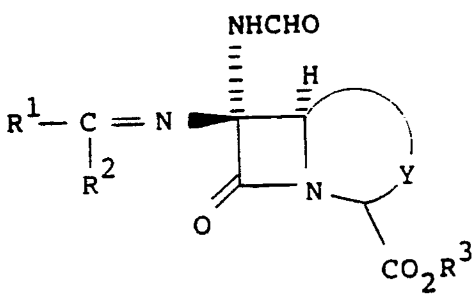

(I)

wherein $R^1$, $R^2$, $R^3$ and Y are as defined in Claim 1; which process comprises reacting a compound of formula (II) or a salt thereof:

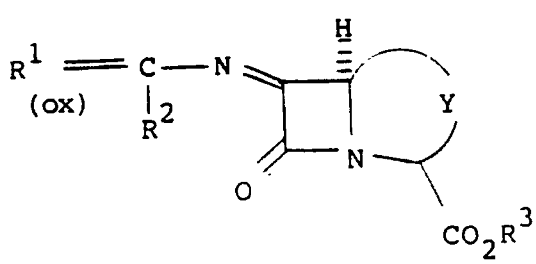

(II)

wherein $R^2$, $R^3$ and Y are as defined for formula (I), $R^1{}_{(ox)}$ is a quinone group corresponding to an oxidised form of the group $R^1$ as hereinbefore defined, and any reactive groups may be protected; with either

(a) a nucleophilic derivative of formamide;

or

(b) ammonia or a protected form, thereof, and thereafter with a formylating agent;

and thereafter if necessary, after step (a) or step (b), carrying out one or more of the following steps:

(i) removing any protecting groups;

(ii) converting the group $R^3$ into a different group $R^3$;

(iii) converting one group Z into a different group Z;

(iv) converting the product into a salt.

7. A process as claimed in Claim 6 in which Y is

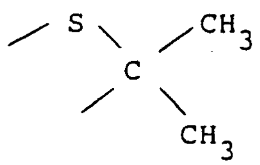

,

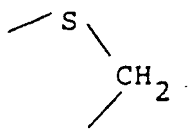

, or

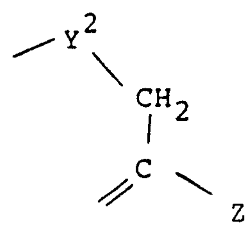

wherein $Y^2$ is oxygen, sulphur or $CH_2$ and Z is as defined in Claim 1.

8. A process as claimed in Claim 6 or Claim 7 wherein the group $R^1{}_{(ox)}$ has one of the subformulae (f)-(j):

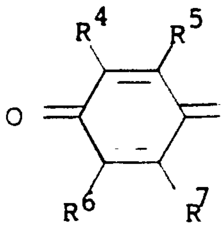

(f)

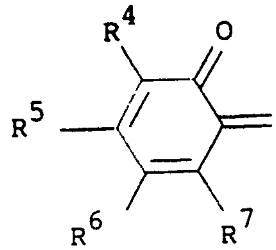

(g)

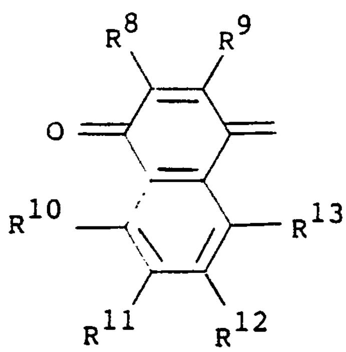

(h)

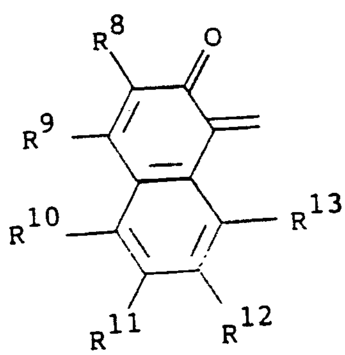

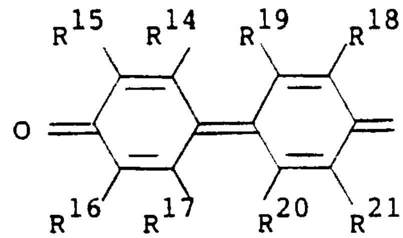

(i) (j)

wherein $R^4$-$R^{21}$ are groups as defined with respect to formulae (a) - (e) in Claim 3.

9. A process as claimed in any one of Claims 6 to 8 wherein $R^1$ is 3,5-di-t-butyl-4-hydroxyphenyl and the group $R^1_{(ox)}$ has the formula:

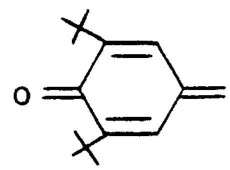

10. A process as claimed in any one of Claims 6 to 9 wherein the reaction is carried out with N,N-bis-trimethylsilylformamide.

11. A process for the preparation of a β-lactam of the formula (IV) or a salt thereof:

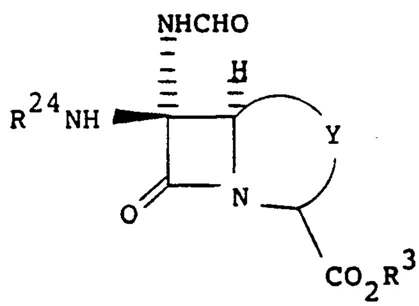

(IV)

wherein $R^3$ and Y are as hereinabove defined and $R^{24}$ is hydrogen or a group $R^{23}$ CO in which $R^{23}$ is a group found in antibacterially active penicillins or cephalosporins; which process comprises treating a compound of the formula (I) as defined in Claim 1, wherein any reactive groups may be protected, with a reagent capable of cleaving the C=N bond and thereafter if necessary carrying out one or more of the following steps:

i) converting the group $R^{24}$ into a different group $R^{24}$
ii) converting the group $R^3$ into a different group $R^3$;
iii) converting one group Z into a different group Z;
iv) removing any protecting groups;
v) converting the product into a salt.

12. A process as claimed in Claim 11 in which the reagent which cleaves the C=N bond is p-toluene sulphonic acid.

Claims for the following Contracting State: ES

1. A process for the preparation of a compound of formula (I) or a salt thereof:

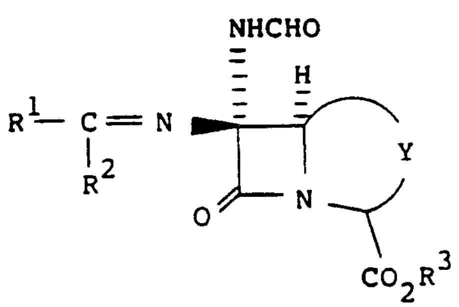

(I)

wherein $R^1$ is a group of the formula:

$HO-[X]_n-$

in which X is an optionally substituted monocyclic or fused polycyclic aromatic ring system in which the

attached hydroxy group is conjugated with the group:

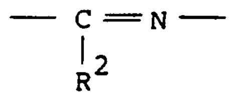

and n is 1 or 2; $R^2$ is hydrogen, $C_{1-6}$ alkyl or aryl $C_{1-6}$ alkyl; $R^3$ is hydrogen or a carboxy protecting group and Y is:

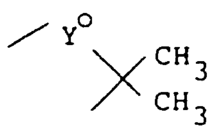

,

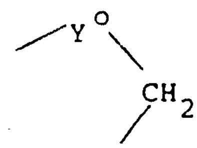

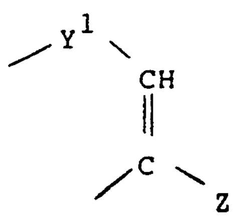

or

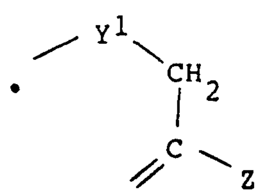

;

wherein $Y^0$ is sulphur, SO or $SO_2$, $Y^1$ is oxygen, sulphur, SO, $SO_2$ or $-CH_2-$ and Z represents hydrogen, halogen, $C_{1-4}$ alkoxy, $-CH_2Q$ or $-CH=CH-Q$ wherein Q represents hydrogen, halogen, hydroxy, mercapto, cyano, carboxy, carbamoyloxy, carboxylic ester, $C_{1-4}$ alkyloxy, acyloxy, aryl, a heterocyclyl group bonded via carbon, a heterocyclylthio group, or a nitrogen containing heterocyclic group bonded via nitrogen; which process comprises reacting a compound of formula (II) or a salt thereof:

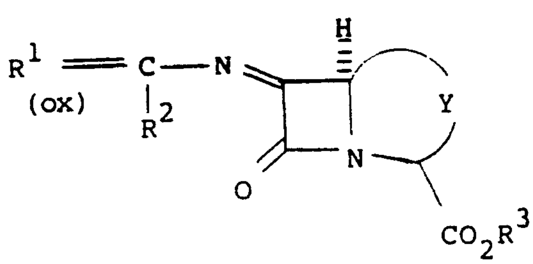

(II)

wherein $R^2$, $R^3$ and Y are as defined for formula (I), $R^1_{(ox)}$ is a quinone group corresponding to an oxidised form of the group $R^1$ as hereinbefore defined, and any reactive groups may be protected; with either

(a) a nucleophilic derivative of formamide;

or

(b) ammonia or a protected form thereof, and thereafter with a formylating agent;

and thereafter if necessary, after step (a) or step (b), carrying out one or more of the following steps:

(i) removing any protecting groups;

(ii) converting the group $R^3$ into a different group $R^3$;

(iii) converting one group Z into a different group Z;

(iv) converting the product into a salt.

2. A process as claimed in Claim 1 in which Y is

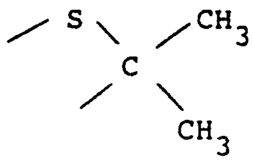 , 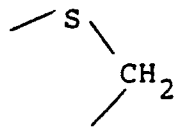 , or 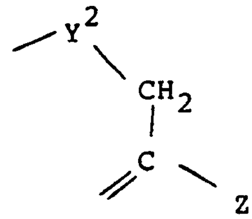

wherein $Y^2$ is oxygen, sulphur or $CH_2$ and Z is as defined in Claim 1.

3. A process as claimed in Claim 1 or Claim 2 wherein the group $R^1_{(ox)}$ has one of the subformulae (f)-(j):

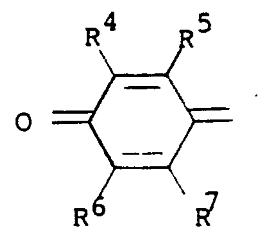

(f)

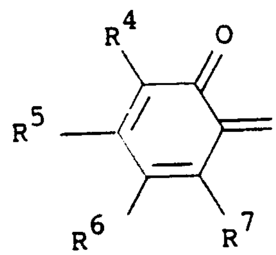

(g)

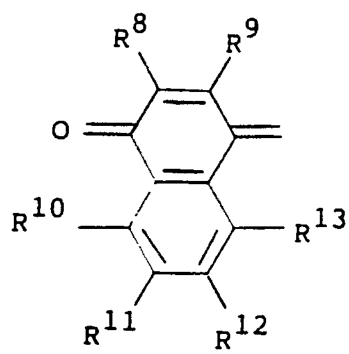

(h)

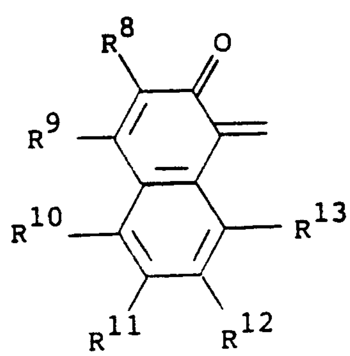

(i)

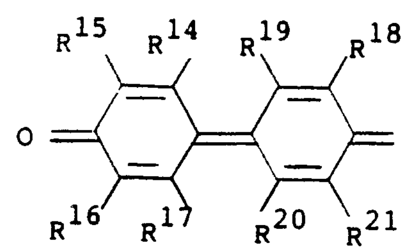

(j)

wherein $R^4$-$R^{21}$ are the same or different and each is hydrogen, halogen, alkoxy, alkylthio, cyano, nitro, carboxy, alkoxycarbonyl, carbamoyl, alkylcarbonyl, amino, mono-alkylamino, di-alkylamino, aryl, heterocyclyl, or an unsubstituted or substituted alkyl group.

4. A process as claimed in any one of Claims 1 to 3 in which $R^2$ is hydrogen.

5. A process as claimed in any one of Claims 1 to 4 in which the compound of formula (II) is formed in situ and reacted without isolation.

6. A process as claimed in any one of Claims 1 to 5 wherein $R^1$ is 3,5-di-t-butyl-4-hydroxyphenyl and the group $R^1_{(ox)}$ has the formula:

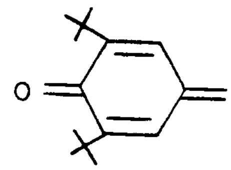

7. A process as claimed in any one of Claims 1 to 6 wherein the reaction is carried out with N,N-bis-trimethylsilylformamide.

8. A process for the preparation of a β-lactam of the formula (IV) or a salt thereof:

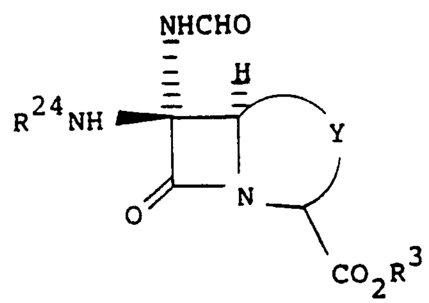

(IV)

wherein $R^3$ and Y are as defined with respect to formula (I) in Claim 1 and $R^{24}$ is hydrogen or a group $R^{23}$ CO in which $R^{23}$ is a group found in antibacterially active penicillins or cephalosporins; which process comprises treating a compound of the formula (I) as defined in Claim 1, wherein any reactive groups may be protected, with a reagent capable of cleaving the C = N bond and thereafter if necessary carrying out one or more of the following steps:

i) converting the group $R^{24}$ into a different group $R^{24}$

ii) converting the group $R^3$ into a different group $R^3$;

iii) converting one group Z into a different group Z;

iv) removing any protecting groups;

v) converting the product into a salt.

9. A process as claimed in Claim 8 in which the reagent which cleaves the C = N bond is p-toluene sulphonic acid.

10. A process as claimed in Claim 8 or Claim 9 for the preparation of 7β-amino-3-chloromethyl-7α-formamidoceph-3-em-4-carboxylic acid or a salt or ester thereof.